# EUROPÄISCHE PATENTSCHRIFT

(11) **EP 4 135 604 B1**
(45) Veröffentlichungstag und Bekanntmachung des Hinweises auf die Patenterteilung: **19.06.2024**
(21) Anmeldenummer: 22731118.0
(22) Anmeldetag: 23.05.2022
(51) Int. Cl.: A61B 17/28, A61B 17/3201

(54) **MEDIZINISCHES INSTRUMENT MIT REINIGUNGSOPTIMIERTER FEDEREINHEIT**
MEDICAL INSTRUMENT HAVING A SPRING UNIT OPTIMISED FOR CLEANING
INSTRUMENT MÉDICAL À UNITÉ DE RESSORT OPTIMISÉE DESTINÉ AU NETTOYAGE

(30) Priorität: 26.05.2021 DE 102021113518
(43) Veröffentlichungstag der Anmeldung: 22.02.2023
(73) Patentinhaber: Aesculap AG, 78532 Tuttlingen (DE)
(72) Erfinder: MORALES, Pedreo, 78532 Tuttlingen (DE); WEISSHAUPT, Dieter, 78194 Immendingen (DE)
(74) Vertreter: Winter, Brandl - Partnerschaft mbB
(86) Internationale Anmeldenummer: PCT/EP2022/063925
(87) Internationale Veröffentlichungsnummer: WO 2022/248414

(56) Entgegenhaltungen:
- CN-A- 110 772 298
- CN-A- 110 785 139
- DE-U1-202009 001 809
- US-A1- 2004 254 428
- US-A1- 2010 222 800

## Beschreibung

### Technisches Gebiet

Die vorliegende Offenbarung betrifft ein medizinisches, insbesondere chirurgisches, Instrument, vorzugsweise ein manuell betätigbares Handinstrument, mit zumindest zwei (einem ersten und einen zweiten) Griffelementen, die schwenkbar zueinander gelagert sind, und einer Federeinheit/Federelastische-Baugruppe, welche zwei Feder-Endabschnitte (an den Federenden) aufweist, die jeweils mit einem der beiden Griffelementen verbunden sind, so dass bei Verschwenken zumindest eines der beiden Griffelemente aus einer Grundstellung (entgegen einer elastischen Kraft der Federeinheit) ein Zurückschwenken in die Grundstellung mittels der Federeinheit durchführbar/realisierbar/bewerkstelligbar ist.

### Stand der Technik

Bei medizinischen bzw. chirurgischen (Hand-)Instrumenten kommen aktuell Rückstellfedern zum Einsatz, die als Blattfedern ausgebildet sind und an einer Branche bzw. einem Griffelement über einen großen Bereich flächig kontaktierend aufliegen. Sowohl die Blattfeder als auch das Griffelement weisen dabei einander zugewandte großflächige, stetige Oberflächen auf, die sich, ausgehend von der gemeinsamen Kontaktauflagefläche, kontinuierlich voneinander entfernen. Man kann auch sagen, dass die Blattfeder und das Griffelement im Abschnitt der Befestigung eine teilkreis-/bogenförmige Kontur mit unterschiedlichen Radien aufweisen und in Folge sukzessiv einen Abstand zwischen sich (von null an) erhöhen. Während also eine Teiloberfläche der Blattfeder mit einer Teiloberfläche des Griffelements flächig in direktem Kontakt stehen, entfernen sich außerhalb die beiden Oberflächen voneinander und bilden einen sehr kleinen Spalt zwischen sich, der erstallmählich größer wird. In Folge lassen sich die medizinischen Instrumente im Bereich der Kontaktauflagefläche nur sehr schwer reinigen und sterilisieren. Zudem ist die Stelle der Auflage von Rückstellfeder und Griffelement einer Kontaktkorrosion bzw. einer Bimetall-Korrosion ausgesetzt.

Die DE 10 2017 114 260 A1 offenbart beispielsweise ein medizinisches Handinstrument, bei dem eine einteilige Rückstellfeder an einem Griffelement mittels Formschluss angebunden ist. Nachteilig an diesem Instrument ist jedoch, dass aufwendig gefräste Aufnahmen an der Branche bzw. Griffelement bereitgestellt werden müssen. Zudem wird eine einteilige Rückstellfeder vorgesehen, so dass eine linear ansteigende hohe Federkraft bei einem Schließen des Handinstruments entsteht. Diese Federkraft wirkt einer durch einen Nutzer, etwa einem Chirurgen, aufzubringenden Kraft entgegen, insbesondere in einer Schließstellung, und mindert so eine maximal manuell aufbringbare Kraft. Ferner liegt ein hoher Verformungsgrad der Feder vor, so dass eine erhöhte Gefahr eines Brechens der Rückstellfeder im Dauereinsatz besteht.

Die US 2004/254428 A1 offenbart ein medizinisches Instrument mit zwei gegeneinander verschwenkbaren Griffelementen, welche durch einen Federmechanismus gegeneinander vorgespannt sind. Diese Federn liegen flächig auf den Innenseiten der Griffelemente auf.

Ferner offenbart die CN 110785139 A ein medizinisches Handinstrument, bei dem eine einteilige Rückstellfeder an einem Griffelement mittels Formschluss angebunden ist.

### Zusammenfassung

Die vorliegende Erfindung wird im Hauptanspruch definiert, Die weiteren Ausführungsformen werden in den Nebenansprüchen definiert. Es sind die Aufgaben und Ziele der vorliegenden Offenbarung, die Nachteile aus dem Stand der Technik zu vermeiden oder wenigstens zu mindern und insbesondere ein medizinisches, insbesondere chirurgisches, Instrument zur Verfügung zu stellen, das eine außerordentlich gute Reinigbarkeit bietet, einfach und kostengünstig in der Herstellung ist und dennoch gleichzeitig eine Kraft für ein Verschwenken der Griffelemente über einen großen, insbesondere gesamten, Wegbereich des Instruments konstant hält. Ferner soll das Instrument insbesondere klein aufbauen, sicher sein, ein einfaches Design haben, gut handhabbar sein und eine Wartung, insbesondere einen Austausch der Federeinheit, vereinfachen.

Die Aufgaben und Ziele hinsichtlich eines medizinisches Instruments werden durch die Merkmale des Anspruchs 1 gelöst.

Die vorliegende Offenbarung betrifft folglich ein medizinisches Instrument mit zwei relativ zueinander schwenkbaren Griffelementen (Griffbügeln, Griffhebeln, Hebelarmen) und einer Federeinheit bzw. federelastischen Baugruppe, die zwischen den Griffelementen angeordnet ist. Die vorzugsweise U-oder V-förmige Federeinheit weist zwei (Feder-)Enden auf, deren sich unmittelbar anschließender Bereich als Endabschnitt bezeichnet wird. Insbesondere bildet das Federende den Feder-Endabschnitt. Jedes der beiden Feder-Endabschnitte ist jeweils mit einem entsprechenden/zugehörigen der zwei Griffelemente befestigt, so dass bei Verschwenken zumindest eines der beiden Griffelemente aus einer Grundstellung ein Zurückschwenken in diese Grundstellung realisiert wird. Das bedeutet, wenn eines der beiden Griffelemente von einem Anwender des Instruments manuell verschwenkt wird, kann die Federeinheit das verschwenkte Griffelement wieder in seine Grundstellung zurückschwenken, sobald der Anwender das verschwenkte Griffteil loslässt. Vorzugsweise weist das Instrument gegenüber einem Scharnier, welches die Griffelemente schwenkbar verbindet, Lastarme auf. Das medizinische Instrument kann auch so ausgebildet werden, dass bei der Anwendung des Instruments beide Griffelemente verschwenkt werden. Ist nur ein Griffelement dafür vorgesehen verschwenkt zu werden, dient das andere Griffelement beim Verschwenken nur als Gegenlager für die Hand des Anwenders. Insbesondere weist das medizinische Instrument einen über den proximalen Griffabschnitt betätigbaren distalen Wirkabschnitt wie einen Klemmabschnitt (Klemm-/Fassbranchen) oder Schneidabschnitt (Schneidklingen) etc. auf.

Gemäß der vorliegenden Offenbarung wird also bei dem Instrument eine solche Federeinheit vorgesehen, die dafür angepasst ist bei Verschwenken der Griffelemente eine in etwa gleichbleibende Kraft/Federkraft/Rückstellkraft bereitzustellen, insbesondere über einen gesamten Schließweg des Instruments. Vorzugsweise wird bei dem Instrument zur Realisierung eine zumindest zweiteilige Federeinheit vorgesehen, die zumindest einen ersten Federabschnitt, insbesondere ersten Feder-Schenkel, und einen (separaten) zweiten Federabschnitt, insbesondere zweiten Feder-Schenkel, aufweist, die miteinander verbunden, insbesondere zumindest teilbeweglich miteinander gekoppelt, sind. Wenn die beiden Schenkel bzw. Griffelemente von der Grundstellung aus zusammengedrückt werden, kann hierdurch eine Federkraft der Federeinheit (bei Verschwenken in eine Schließstellung), insbesondere über den gesamten Wegbereich des Schließens, gleichmäßig und homogen gehalten werden. Ein linearer Anstieg der Federkraft unterbleibt. Diese spezielle Gestaltung unterstützt einen Nutzer des Instruments und erhöht letztlich auch eine potentiell maximal aufbringbare Schließkraft und wirkt einer Ermüdung des Nutzers entgegen.

Zudem wird die Federeinheit noch speziell angepasst an die Griffelemente verbunden. Konkret wird zumindest ein Teil der Federeinheit an zumindest einem Feder-Endabschnitt, insbesondere Federende, an oder über einen podestartigen/podestförmigen Vorsprung bzw. einem Podest an dem zugehörigen Griffelement des Instruments angebunden, insbesondere befestigt. Hierdurch wird im Bereich des podestförmigen Vorsprunges und damit des Feder-Endabschnitts ein definierter (Mindest-)Abstand zwischen dem Abschnitt der Feder, insbesondere eines gesamten Feder-Schenkels, und dem zugehörigen Griffelement realisiert. Der sich so ausbildende Spalt stellt zumindest in der Grundstellung ein Mindestspaltmaß zwischen dem Feder-Schenkel und dem Griffelement sicher, so dass eine gute Reinigung des Instruments gewährleistet ist. Auch kann über den podestförmigen Vorsprung ein geometrischer Übergang zwischen Federeinheit und Griffelement definiert gestaltet werden, etwa durch Abrundung von Kanten, um eine Reinigbarkeit weiter zu verbessern. Eine Herstellung des Instruments wird deutlich vereinfacht.

Mit anderen Worten wird zumindest also insbesondere ein Federabschnitt, insbesondere Feder-Schenkel, der Federeinheit über einen dazwischenliegenden/zwischengeschalteten podestförmigen Vorsprung/Absatz/Sockel/Abstandshalter/Bänkchen an dem zugehörigen/entsprechenden Griffelement starr befestigt, der insbesondere eine (einzige) definierte Kontaktfläche ausbildet, über die der Federabschnitt, insbesondere Feder-Schenkel, und das zugehörige Griffelement in zumindest der Grundstellung miteinander in Kontakt stehen, so dass diese zwischen sich einen Spalt mit einer Mindestgröße bzw. einen vordefinierten Abstand aufweisen. Insbesondere ist die Federeinheit nur über den bzw. die podestförmigen Vorsprünge mit den Griffelementen verbunden und steht mit diesen in Kontakt. Die Reinigbarkeit und Herstellung des Instruments wird verbessert.

Mit noch anderen Worten ist die Federeinheit dafür angepasst bei Verschwenken der Griffelemente eine im Wesentlichen gleichbleibende Federkraft bereitzustellen. Insbesondere ist die Federeinheit zumindest zweiteilig ausgebildet und weist einen ersten Teil, insbesondere einen ersten Feder-Schenkel, und einen zweiten Teil, insbesondere einen zweiten Feder-Schenkel auf, um eine gleichmäßige Federkraft sicherzustellen. Ferner ist an zumindest einem der beiden Feder-Endabschnitte und/oder an zumindest einem der beiden Griffelemente ein podestförmiger Vorsprung ausgebildet/angeformt/ausgeformt, über den der Feder-Endabschnitt mit dem zugehörigen Griffelement starr verbunden ist, so dass in zumindest der Grundstellung der zumindest eine Feder-Endabschnitt gegenüber dem zugehörigen Griffelement erhöht/versetzt angeordnet ist und einen Spalt zwischen sich ausbildet bzw. einen durch den podestförmigen Vorsprung bedingten Abstand zu dem Griffelement aufweist.

Noch anders ausgedrückt weist das medizinische Instrument, vorzugsweise der Zangen- oder Stanzenbauart, also zumindest zwei schwenkbar zueinander gelagerte Branche/Instrumententeile/Schenkel auf, die einen, in Instrumentenlängsrichtung gesehen, proximalen Griffabschnitt/Betätigungsabschnitt aufweisen. Das Instrument hat eine federelastische Rückstelleinheit/Federeinheit, insbesondere in Form einer Rückstellfeder, die zwischen der ersten Branche und der zweiten Branche, vorzugsweise im Bereich des Griffabschnitts, vorgesehen und/oder an/ausgeformt ist, derart, dass sie die erste Branche und die zweite Branche in einer Grundstellung halten bzw. in diese vorspannen. Zwischen zumindest der ersten Branche und einem ersten Feder-Abschnitt der Federeinheit ist ein podestförmiger Vorsprung/Absatz/Sockel vorgesehen über den sie miteinander verbunden sind und, der an der ersten Branche und/oder an dem ersten Feder-Abschnitt ausgeformt ist. Alternativ oder zusätzlich kann zwischen der zweiten Branche und dem zweiten Feder-Abschnitt ebenso ein podestförmiger Vorsprung/Absatz/Sockel vorgesehen sein über den sie miteinander verbunden sind, der an der zweiten Branche und/oder an dem zweiten Feder-Abschnitt ausgebildet ist. Die Zwischenräume lassen sich sehr gut reinigen.

Der Begriff Grundstellung definiert eine Stellung der verschwenkbaren Griffelemente zueinander, in welche sie sich ohne äußere (manuelle) Krafteinwirkung, nur bedingt durch die Federeinheit, begeben. Diese Grundstellung kann insbesondere eine (geometrisch bedingte) Maximalöffnungsstellung des Instruments sein.

Der Begriff podestförmiger Vorsprung definiert eine Geometrie ähnlich einer Plattform, die dazu geeignet ist eine zweite Fläche (erhöhte Fläche) gegenüber einer ersten Fläche (Grundfläche, Umgebungsfläche, Basisfläche) in einer Richtung im Wesentlichen orthogonal auf die erste Fläche an dieser Stelle definiert zu erheben / zu erhöhen / abzusetzen / zu versetzen. Man kann auch sagen, dass durch den podestförmigen Vorsprung eine Abstufung (mit individueller Form einer Stufe, insbesondere Blockstufe) erreicht wird.

Der Begriff Feder-Endabschnitt definiert einen Abschnitt des Federendes. Insbesondere kann das Federende der Feder-Endabschnitt sein.

Vorteilhafte Ausführungsformen sind in den Unteransprüchen beansprucht und werden nachfolgend erläutert.

Gemäß einem Aspekt der vorliegenden Offenbarung kann Federeinheit zweiteilig in Form von zwei separaten Blattfedern oder zwei Federstahldrähten ausgebildet sein, die distal miteinander verbunden sind, insbesondere über eine distale Gabel-Nasen-Verbindung oder eine distale Kugel-Pfannen-Verbindung. Die erste Blattfeder bildet den ersten Federschenkel und die zweite Blattfeder den zweiten Federschenkel. Durch die zweiteilige Ausführung wird eine Anzahl an Komponenten gering gehalten und eine Spaltbildung in Kontaktbereichen minimiert. Die Blattfedern lassen sich sowohl kostengünstig und einfach herstellen als auch montieren. Insbesondere weisen die beiden Blattfedern in einer Draufsicht auf das Instrument bzw. in einer Seitenansicht der Blattfedern eine konvexe Bogenform auf, die an der distalen Verbindungsstelle spitz/pfeilförmig zuläuft.

Gemäß einem weiteren Aspekt der Offenbarung kann der podestförmige Vorsprung eine stirnseitige Kontaktauflagefläche ausformen, an der bzw. über die der Feder-Endabschnitt bzw. die Federeinheit mit dem zugehörigen Griffelement befestigt ist. Wenn der Feder-Endabschnitt bzw. die Federeinheit und das zugehörige Griffelement separate Bauteile sind, wird durch die Kontaktauflagefläche eine definierte Schnittstelle bzw. Verbindungsstelle geschaffen, auf die der Feder-Endabschnitt bzw. eine Oberfläche des Griffelements aufliegt. Der Begriff stirnseitig bedeutet, dass die Fläche an der erhöhten Seite (also der gegenüber der Grundfläche beabstandeten Fläche) ausgeformt ist. Dabei kann die Grundfläche die maßgebliche Oberfläche des Feder-Endabschnitts sein, wenn der podestförmige Vorsprung an dem Feder-Endabschnitt ausgebildet ist, und/oder die maßgebliche Oberfläche (um den podestförmigen Vorsprung herum) des Griffelements sein, wenn der podestförmige Vorsprung an dem Griffelement angeformt ist. Die Kontaktauflagefläche selbst kann dabei unterschiedliche Flächenformen annehmen, also auch etwa leicht gewölbt (konvex oder konkav) oder geschwungen oder wellenförmig sein.

Vorzugsweise kann der podestförmige Vorsprung blockartig ausgebildet sein mit einer Block-Grundfläche, die sich senkrecht zur maßgeblichen Grundfläche in Richtung einer Höhe des podestförmigen Vorsprungs erstreckt und stirnseitig die Kontaktauflagefläche ausbildet. Die Höhe des podestförmigen Vorsprungs ist insbesondere senkrecht zu einer Griffelementenlängsachse (an der Stelle) bzw. zu einer Federn-Endabschnitt-Längsachse (an dieser Stelle) zu sehen. Insbesondere kann die Block-Grundfläche eine im Wesentlichen rechteckige Grundform aufweisen. Alternativ kann die Grundform auch eine ovale Grundform aufweisen. Vorzugsweise ist die Außenkontur der Block-Grundfläche konvex, so dass keine Hinterschnitte an Seitenflächen vorhanden sind. Die Seitenflächen erstrecken sich um die Kontaktauflagefläche bzw. die Block-Grundfläche herum zumindest in Richtung der Höhe des podestförmigen Vorsprungs, bilden also die Seitenflächen des podestförmigen Vorsprungs.

Gemäß einem weiteren Aspekt der Offenbarung kann zumindest einer der beiden Feder-Endabschnitte über den podestförmigen Absatz mit dem Griffelement mittels einer formschlüssigen Verbindung, insbesondere Schraubverbindung und/oder stoffschlüssigen Verbindung, insbesondere mittels Verkleben und/oder Verschweißen und/oder Verlöten, verbunden sein. Im Falle einer Schraubverbindung wird eine kostengünstige Montage realisiert welche die Federeinheit sicher und langlebig mit den Griffelementen verbindet und die Federeinheit kann leicht demontiert und ausgetauscht werden. Im Falle einer Verklebung oder Verschweißung oder Verlötung können Spalte, die im Bereich der Verbindung zwangsläufig entstehen, hermetisch geschlossen werden und eine geschlossene Oberfläche sichergestellt werden. Dies unterstützt eine Reinigung und Sterilisation.

Im Falle der Schraubverbindung kann insbesondere die Schraube von Seiten des Feder-Endabschnitts in Richtung des zugehörigen Griffelements in eine komplementäre Sackloch/Sacklochbohrung mit Innengewinde eingeschraubt sein, so dass das Griffelement an einer äußeren Grifffläche kein Bohrloch aufweist. Mit anderen Worten wird eine Schraube, insbesondere senkrecht auf, und durch den Feder-Endabschnitt hindurch in das Griffelement eingeschraubt. Wenn der podestförmige Vorsprung an dem Griffelement ausgeformt ist, kann die Schraube in ein in einem Sackbohrloch vorgesehenen Innengewinde des Vorsprungs eingeschraubt werden. So wird außenseitig an der Grifffläche/Angreiffläche eine scharfe Kante vermieden, welche eine Gefahrenstelle eines Aufreißens eines Operationshandschuhs darstellt. Ebenfalls wird eine ebene Oberfläche der äußeren Grifffläche nicht unterbrochen und die Oberfläche geschlossen gehalten. Alternativ oder zusätzlich kann vorzugsweise die Schraube auch von Seiten des Griffelements in Richtung des Feder-Endabschnitts eingeschraubt sein. Insbesondere kann der podestförmige Vorsprung an dem Feder-Endabschnitt ausgeformt sein und die Schraube in dem Vorsprung eingeschraubt sein, so dass diese nicht durch den Feder-Endabschnitt hindurchsteht und eine abgewandte Oberfläche des Feder-Endabschnitts nicht durchbrochen bzw. geschlossen ist. Mit dieser Alternative der Schraubverbindung wird eine Montage als auch ein Austausch der Federeinheit vereinfacht.

Gemäß einer weiteren Ausführungsform kann der podestförmige Vorsprung eine Höhe von mindestens 1mm, bevorzugt von mindestens 2mm, besonders bevorzugt von mindestens 4mm aufweisen. Die Höhe definiert einen Abstand zwischen einem Fußpunkt (an der Grundfläche) des Griffelements und gewissermaßen einem Fußpunkt des Feder-Endabschnitts beim podestförmigen Vorsprung. Dieser Abstand wird insbesondere senkrecht zu einer Instrumentenlängsachse des Griffelements an der Stelle des podestförmigen Vorsprungs gemessen. Insbesondere ist die Höhe der Abstand auf der distalen Seite des podestförmigen Vorsprungs, also der Seite zur Lagerung hin. Insbesondere ist die Höhe ein Abstand zwischen der stirnseitigen Kontaktauflagefläche des podestförmigen Vorsprungs und seines Fußpunkts bzw. einer der Federeinheit zugewandten Grundfläche/(Basis-)Oberfläche des Griffelements, sodass ein sich ausbildender Spalt zwischen dem Griffelement und dem Feder-Endabschnitt in zumindest der Grundstellung des Instruments ebenfalls mindestens diese Höhe hat. Dieses Mindest-Spaltmaß bzw. diese Höhe stellt eine ausreichend gute Reinigbarkeit sicher.

Insbesondere kann der podestförmige Vorsprung eine Höhe von maximal 5mm aufweisen, so dass bei einer Schließbewegung (von der Grundstellung aus) die beiden Feder-Endabschnitte sich selbst in einer (maximalen) Schließstellung nicht kontaktieren.

Vorzugsweise kann eine Kontaktauflagefläche des podestförmigen Vorsprungs mindestens 5mm² (mm^2/Quadratmillimeter) und/oder höchstens 1cm² (cm^2/Quadratzentimeter) betragen und/oder plan ausgebildet sein. Mit dem Mindestflächenmaß wird eine ausreichende Stabilität der Verbindung von Griffelement und Feder-Endabschnitt sichergestellt. Mit dem Höchstflächenmaß wird dahingehend Rechnung getragen, dass ein Baumaß kompakt bleibt und eine ausreichende Federkraft verbleibt.

Gemäß einem Aspekt der Offenbarung kann ein Winkel zwischen einer dem zugehörigen Griffabschnitt zugewandten Seite des Feder-Endabschnitts, die insbesondere auf der Kontaktauflagefläche aufliegt, und einer Seitenfläche des podestförmigen Vorsprungs mindestens 20°, bevorzugt mindestens 40°, besonders bevorzugt über 65° und ganz besonders bevorzugt 90° betragen. Mit diesem Mindestwinkel wird ein Spalt ausreichend stumpf und nicht zu spitz zulaufend gestaltet. Insbesondere kann alternativ oder zusätzlich auch ein Winkel zwischen der der Federeinheit zugewandten Grundfläche/(Basis-)Oberfläche des Griffelements und einer Seitenfläche des podestförmigen Vorsprungs höchstens 140°, bevorzugt höchstens 110°, besonders bevorzugt 90° betragen. Auch kann ein Abschnitt der Seitenwand eine bogenförmige zum Griffelement und/oder zum Feder-Endabschnitt, insbesondere zur stirnseitigen Kontaktfläche, hin auslaufende Form/Phase aufweisen, um eine Kante zu vermeiden und die Reinigbarkeit zu erhöhen. Mit anderen Worten kann zumindest eine das Griffelement oder den Feder-Endabschnitt berührende Kante des podestförmigen Vorsprungs angefast sein (eine Voute).

Gemäß einem weiteren Aspekt der vorliegenden Offenbarung kann der podestförmige Vorsprung ein Ausrichtungselement, insbesondere in Form eines vorspringenden Anschlags oder eines Pins aufweisen, um den Feder-Endabschnitt gegenüber dem Griffelement, wenn separat ausgebildet und auf der Kontaktauflagefläche aufliegend, auszurichten.

Insbesondere verlaufen die einander zugewandten Außenkonturen und/oder Oberflächen von zumindest einem der beiden Griffelemente und dem zugehörigen Feder-Schenkel im Bereich des podestförmigen Vorsprungs im Wesentlichen parallel zueinander. Insbesondere verlaufen sie distal des podestförmigen Vorsprungs parallel zueinander. Mit anderen Worten verlaufen die Grifflängsachse und die Feder-Schenkel-Längsachse im Bereich distal des podestförmigen Vorsprungs im Wesentlichen parallel zueinander, insbesondere bis zu einer Länge von 30%, bevorzugt bis einer Länge von 50% der Gesamtlänge des Feder-Schenkels. Insbesondere wenn eine Blattfeder als Feder-Schenkel Verwendung findet, weisen Blattfeder und Griffelement in zumindest einer Stellung abschnittsweise einen annähernd gleichen bzw. parallelen Verlauf auf.

Bei einer weiteren Ausführungsform kann der podestförmige Vorsprung eine Länge (in Richtung der Griffelementlängsachse bzw. in Richtung Feder-Endabschnitt-Längsachse) von mindestens 5mm, bevorzugt mindestens 10mm, aufweisen. Diese Mindestlänge erzielt eine ausreichende mechanische Festigkeit oder Kontaktauflagefläche, um ein Verbiegen der Federeinheit oder Lösen von dem Griffelement zu verhindern.

Insbesondere weist der podestförmige Vorsprung eine, insbesondere distale, Seitenfläche, auf, die senkrecht auf die Kontaktauflagefläche steht.

In einer Ausführungsform kann zumindest ein Feder-Schenkel, der podestförmige Vorsprung und das zugehörige Griffelement einstückig miteinander ausgebildet sein.

Vorzugsweise ist der zumindest eine Feder-Endabschnitt derart mit dem zugehörigen Griffelement verbunden, dass die Federeinheit sich nur in der Schwenkebene verformt.

Mit anderen Worten betrifft die vorliegende Offenbarung vorzugsweise ein medizinisches Instrument, dass sich dadurch kennzeichnet, dass eine Federvorrichtung/Federeinheit mit insbesondere zwei Blattfedern, je Branche eine, erhöht auf einem Bänkchen (podestförmigen Vorsprung) montiert sind, vorzugsweise verschraubt. Da die Federeinheit, insbesondere die zwei Blattfedern durch die Bänkchen (podestförmigen Vorsprünge) auf Abstand (zu dem zugehörigen Griffelement) gehalten werden, lässt sich eine gute Reinigbarkeit wie etwa Sterilisierbarkeit erreichen. Insbesondere lassen sich die Zwischenräume sehr gut bzw. problemlos reinigen. Die Kontaktfläche (des einen Griffelements mit dem Federelement) wird auf die Länge des Bänkchens (podestförmigen Vorsprungs) begrenzt. Wenn insbesondere eine zweiteilige Feder zum Einsatz bei dem medizinischen Instrument kommt, hat dies den Vorteil, dass bei einem Schließen des Instruments, insbesondere einer Zange, die Federkraft nicht linear ansteigt. Die Schließkräfte bleiben hierdurch nahezu konstant. Die zweiteilige Feder steht insbesondere distal über eine Federkraft, vorzugsweise durch eine Gabel/Nasenverbindung oder eine Kugel/Pfannenverbindung in Kontakt.

### Kurzbeschreibung der Figuren

Die vorliegende Offenbarung wird nachfolgend anhand bevorzugter Ausführungsformen unter Bezugnahme auf die begleitenden Figuren näher erläutert. Es zeigen:
- Fig. 1: eine Draufsicht auf einen distalen Griffabschnitt eines medizinischen Instruments einer ersten bevorzugten Ausführungsform, bei der eine Federeinheit auf podestförmigen Vorsprüngen, die jeweils an einem Schenkel ausgebildet sind, aufliegt und befestigt ist;
- Fig. 2: eine Draufsicht eines medizinischen Instruments einer zweiten bevorzugten Ausführungsform, bei der an der Federeinheit selbst podestförmige Vorsprünge angeformt sind, die an den Griffelementen aufliegen und befestigt sind;
- Fig. 3: eine Draufsicht auf ein medizinisches Instrument einer dritten bevorzugten Ausführungsform, welche eine Kombination der beiden Ausführungsformen aus Fig. 1 und 2 darstellt;
- Fig. 4: eine detaillierte Teilansicht eines medizinischen Instruments einer weiteren, vierten bevorzugten Ausführungsform, bei der an der Federeinheit der podestförmige Vorsprung ausgebildet ist und mit einer Schraubverbindung von außen von Seiten des Griffelements befestigt ist;
- Fig. 5: eine Draufsicht auf ein medizinisches Instrument einer weiteren, fünften bevorzugten Ausführungsform, bei der die podestförmigen Vorsprünge an der Federeinheit ausgebildet ist welche auf den Griffelementen aufliegen und angeschweißt sind; und
- Fig. 6: eine detaillierte Teilansicht einer Verbindungsstelle eines medizinischen Instruments einer weiteren, sechsten bevorzugten Ausführungsform, bei der die Verbindung zwischen der Federeinheit und dem podestförmigen Vorsprungs des Griffelements stoffschlüssig mittels Klebeverbindung realisiert ist.

Die Figuren sind schematischer Natur und sollen lediglich dem Verständnis der vorliegenden Offenbarung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen

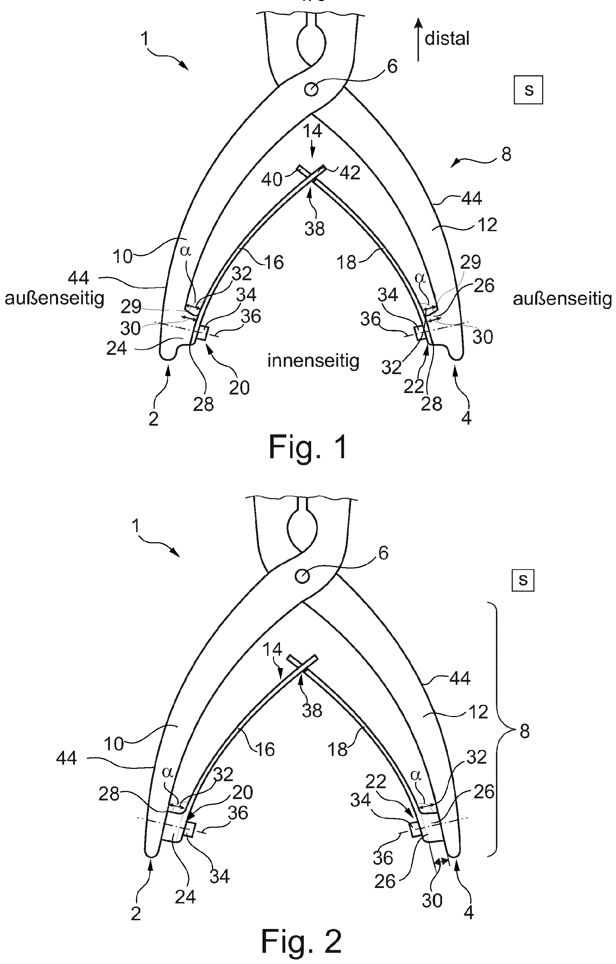

Fig. 1 zeigt in einer Teilansicht ein medizinisches Instrument 1 einer ersten bevorzugten Ausführungsform. Das Instrument 1, in Form eines Handinstruments, ist nach Zangenbauart ausgebildet. Das Instrument 1 weist dafür zwei Hebel 2, 4 auf, welche miteinander über ein Scharnier 6 schwenkbar verbunden sind. So lassen sich die beiden Hebel 2, 4 in einer Schwenkebene S zueinander, ähnlich einer Schere oder Fasszange, verschwenken. Die proximalen Abschnitte der Hebel 2, 4 (proximal des Scharniers 6 und zum Nutzer weisend) bilden dabei einen Griffabschnitt 8 mit entsprechenden Griffelementen bzw. Griffen 10, 12 aus, die im Wesentlichen symmetrisch zueinander gegenüber einer Instrumentenlängsachse (proximal-distal) ausgebildet sind. Auch ist der Griffabschnitt 8 symmetrisch zu der Schwenkebene S gestaltet.

Zwischen dem ersten Griff 10 und dem zweiten Griff 12 ist eine zweiteilige Federeinheit 14 in Form einer zweiteiligen Blattfeder vorgesehen, welche im Wesentlichen V-förmig mit leichter Ausbauchung ausgebildet ist pfeilförmig distal zuläuft und zwei Feder-Schenkel 16, 18 aufweist, welche die Blattfedern bilden. Bei einem Verschwenken zumindest eines der beiden Griffen 10, 12 aus einer Grundstellung des Instruments entgegen einer elastischen Kraft der Federeinheit 14, wird ein Zurückschwenken in die Grundstellung mittels der Federeinheit 14 realisiert. An ihrem jeweiligen freien Feder-Endabschnitten 20, 22 liegen die Feder-Schenkel 16, 18 auf sich gegenüberliegenden/einander zugewandten podestförmigen/podestartigen Vorsprüngen/Absätzen 24, 26 auf, die an einander zugewandten Innenseiten/Innenflächen der Griffe 10, 12 ausgebildet sind. Der erste podestförmige Vorsprung 24 und der erste Griff 10 sowie der zweite podestförmige Vorsprung 26 und der zweite Griff 12 sind dabei einstückig/integral ausgebildet bzw. angeformt. Durch den podestförmige Vorsprung 24, 26 werden die beiden Feder-Endabschnitte 20, 22 definiert gegenüber der Umgebung, hier die Innenseiten bzw. innenliegenden Oberflächen (Grundflächen) der Griffe 10, 12 erhöht und es bildet sich ein Mindestabstand zwischen dem jeweiligen Griff 10, 12 und dem jeweiligen Feder-Schenkel 16, 18 aus.

Durch die spezielle zweiteilige Gestaltung der Federeinheit 14 als Rückstellfeder kann für eine gute Handhabung und Haltbarkeit einerseits eine im Wesentlichen gleichbleibende Federkraft bei einem Verschwenken der beiden Hebel 4, 6 bzw. Griffe 10, 12 in der Schwenkebene S über den gesamten Schließweg bereitgestellt werden und andererseits eine einfache Gestaltung und kostengünstige Herstellung. Durch die spezielle Anbindung des ersten und zweiten Feder-Schenkels 16, 18 über den podestförmigen Vorsprung 24, 26 wird eine gute Reinigbarkeit sowie ebenfalls eine einfache Gestaltung und kostengünstige Herstellung des Instruments 1 gewährleistet.

Konkret liegen die beiden Blattfedern der Federeinheit 14 jeweils flächig auf einer stirnseitigen Kontaktauflagefläche 28 des podestförmigen Vorsprungs 24, 26 auf. Die Kontaktauflagefläche 28 definiert die einzige strukturelle Verbindung der Federeinheit 14 mit jeweils den Griffen 10, 12. Über eine Höhe 30 der podestförmigen Vorsprünge 24, 26, die im Wesentlichen senkrecht auf eine Grifflängsachse steht, kann die Abmessung eines sich zwischen dem Feder-Schenkel 16, 18 und dem Griff 10, 12 ausbildenden Spalts bzw. eine Spaltbreite 32 eingestellt werden. Die Spaltbreite 32 wird von der innenliegenden Oberfläche der Griffe 10, 12 (als Grundfläche) zu der gegenüberliegenden Oberfläche der Feder-Schenkel 16, 18 (erhöhte Fläche; entspricht der Kontaktauflagefläche 28) bemessen. Insbesondere beträgt eine Höhe 3mm und damit auch die in Fig. 1 gezeigte geringste Spaltbreite 32 ebenfalls 3mm.

Um die Blattfedern auch auf den podestförmigen Vorsprüngen 24, 26 fest zu fixieren, sind innen von Seiten der Blattfedern her jeweils eine Schrauben 34 mit Schraubenlängsachse 36 in Richtung der Höhe 30 des Vorsprungs 24, 26 bzw. im Wesentlichen senkrecht auf eine Grifflängsachse bzw. senkrecht zur Kontaktauflagefläche 28 nach außen in ein entsprechendes Innengewinde eines Sacklochs (nicht dargestellt) in dem Griff 10, 12 eingeschraubt. Dies ermöglicht eine einfache Montage und bei Bedarf, etwa zur Wartung oder zum Austausch der Federeinheit 14, auch einer Demontage. Zudem lässt sich das Instrument 1 auch sehr einfach herstellen und ohne großes Vorwissen fehlerfrei montieren.

Die zweiteilige Federeinheit 14 verbindet bzw. koppelt mittels einer distale Feder-Kopplung 38 die beiden Feder-Schenkel 16, 18 miteinander. In dieser ersten Ausführungsform ist die Feder-Kopplung in Form einer Gabel-Nasen-Verbindung gestaltet, bei der der erste Feder-Schenkel 16 eine Nase 40 und der zweite Feder-Schenkel eine Gabel 42 hat. Die Nase 40 steht dabei in die Gabel 42 hinein und wird (leicht) verschieblich und verschwenkbar von dieser gehalten. So lassen sich bei einer Schwenkbewegung der Griffe 10, 12 zueinander, die distalen Enden der Feder gegeneinander verschwenken und es wird eine im Wesentlichen konstante bzw. gleichbleibende Federkraft entlang des gesamten Schließwegs sichergestellt. Alternativ kann natürlich auch eine Kugel-Pfannen-Verbindung statt der Gabel-Nasen-Verbindung verwendet werden. Die Federeinheit 14 hat eine Pfeilspitzenform mit spitz zulaufender distaler Feder-Kopplung 38 und ausbauchenden Feder-Schenkeln 16, 18.

Ein Winkel bzw. Spaltwinkel α zwischen einer Unterseite der Blattfedern (die den Griffen 10, 12 zugewandt ist) und einer sich direkt anschließenden Seitenfläche 29 der podestförmigen Vorsprünge beträgt in dieser Ausführungsform 70°. Auf diese Wiese wird ein ausreichend stumpfer Winkel bereitgestellt. Zudem ist distal und zu Seiten der Kontaktauflagefläche 28 eine leichte Abrundung nach distal vorgesehen, um eine Reinigung zu unterstützen.

Da die Schrauben 34 von innen nach außen zu den Griffen 10, 12 entlang der Schraubenlängsachse 36 in ein Sackloch mit Innengewinde eingeschraubt sind, weist eine außenliegende bzw. äußere Grifffläche 44 eine ebene und geschlossene Oberfläche auf. Hierdurch wird einer Verkeimung und einer Beschädigung eines chirurgischen Handschuhs vorgebeugt, da der Handschuh eine scharfe Kante für ein Aufreißen vermieden wird.

Das in Fig. 2 gezeigte medizinisches Instrument 1 einer zweiten bevorzugten Ausführungsform unterscheidet sich gegenüber der ersten bevorzugten Ausführungsform dahingehend, dass podestförmige Vorsprünge 24, 26 nicht einstückig an den Griffelementen 10, 12 aus- bzw. angeformt sind, sondern an den beiden Feder-Schenkeln 16, 18 der Federeinheit 14. Die Feder-Schenkel 16, 18 sind über ihre podestförmigen Vorsprünge 24, 26, genauer gesagt über die Kontaktauflageflächen 28, auf innenliegende Oberflächen der Griffe 10, 12 flächig aufliegend befestigt. Auch in dieser Ausführungsform werden die Feder-Schenkel 16, 18 mittels Schrauben 34 von Seiten der Feder-Schenkel 16, 18 her in die Griffe 10, 12 eingeschraubt, so dass eine äußere Grifffläche 44 eine geschlossene Oberfläche ohne Öffnungen aufweist. Auf diese Weise können standardisierte Instrumente bzw. Griffelemente verwendet werden, in welche nur eine Sacklochbohrung mit Innengewinde eingebracht wird. Es muss lediglich die Federeinheit 14 an die innenseitigen Oberflächen der Griffe 10, 12 entsprechend angepasst werden.

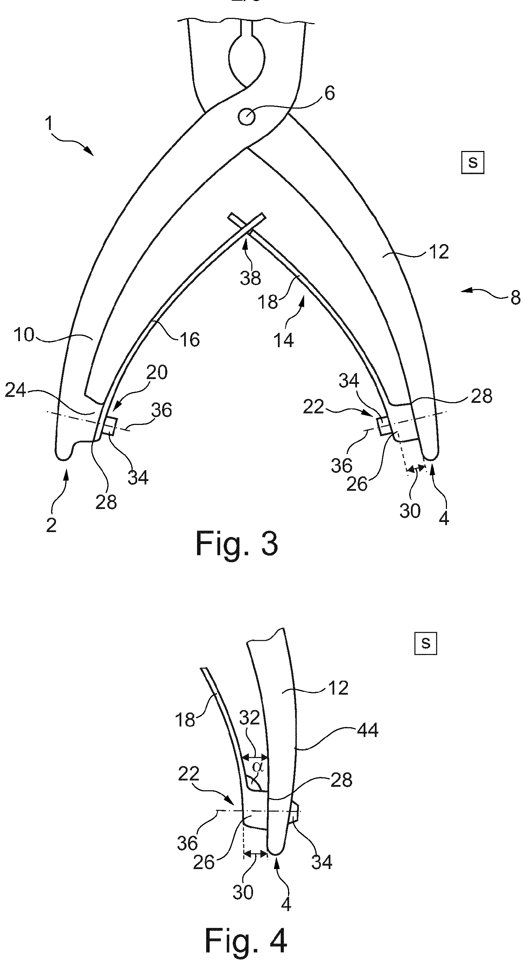

Fig. 3 zeigt eine weitere, dritte Ausführungsform des Instrument 1, bei der eine Kombination der podestförmigen Vorsprünge 28 des ersten Griffs 10 der ersten Ausführungsform und des zweiten Griffs 12 der zweiten Ausführungsform Verwendung findet. Konkret ist einstückig an dem ersten Griff 10 ein podestförmiger Vorsprung 24 ausgeformt, auf dessen Kontaktauflagefläche 28 die Blattfeder bzw. der erste Feder-Schenkel 16 aufliegt und befestigt ist, wohingegen der zweite Griff 12 keinen podestförmigen Vorsprung aufweist, jedoch der zweite Feder-Schenkel 18 den zweiten podestförmigen Vorsprung 26 hat, der einstückig an bzw. mit diesem angeformt ist.

Im Gegensatz zu den ersten drei Ausführungsformen, ist in der Fig. 4 bei einem medizinischen Instrument 1 einer weitere bevorzugten Ausführungsform die Federeinheit 14 mit dem zweiten Griff 12 über eine Schraube 34 verbunden, die von Seiten der äußeren Grifffläche 44 des zweiten Griffelements 12 her (hier beispielhaft nur eine Seite des Instruments 1 zur Veranschaulichung dargestellt) in Richtung des Feder-Endabschnitts 22 in eine in dem podestförmigen Vorsprung 26 ausgebildet Sacklochbohrung mit Innengewinde eingeschraubt ist. Diese Gestaltung hat den Vorteil, dass von außen die Schraube gut einschraubt werden können und dass innenseitig an dem Feder-Schenkel bzw. dem Feder-Endabschnitt eine geschlossene Oberfläche ohne Öffnung vorgesehen ist, um einer Keimbildung vorzubeugen.

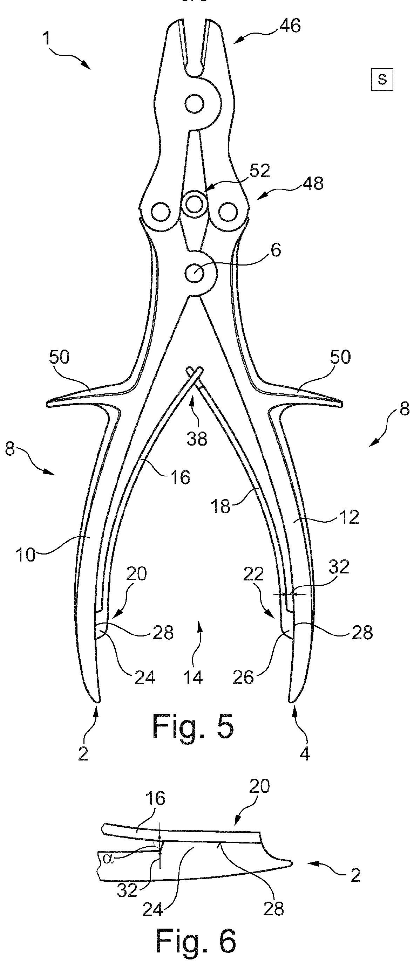

In Fig. 5 ist ein medizinische Instrument 1 einer weiteren bevorzugten Ausführungsform in Form einer Branchenzange mit einer Mehrzahl an Scharnieren bzw. einem mehrgliedrigen Hebelmechanismus 48 und einem distalen Zangenschneidabschnitt 46 ausgebildet. Im Unterschied zu den vorherigen Ausführungsformen sind die beiden an der Federeinheit 14 angeformten podestförmigen Vorsprünge 24, 26 nicht angeschraubt, sondern beidseitig stoffschlüssig an den Griffen 10, 12 befestigt. In der vorliegenden Ausführungsform sind diese an die Griffe 10, 12 angeschweißt um eine dichte und lang haltende Verbindung zwischen den zwei Komponenten zu schaffen. In dieser Ausführungsform ist die Höhe 30 der podestförmigen Vorsprünge 24, 26 und damit auch die Spaltbreite 32 geringer gestaltet, da eine Mindest-Einschraubtiefe nicht gewährleistet sein muss. Dennoch wird durch den podestförmigen Vorsprung 24, 26 ein Mindestabstand in der in Fig. 5 gezeigten Reinigungsstellung sichergestellt, so dass das Instrument 1 sich gut reinigen lässt. Die Reinigungsstellung unterscheidet sich in dieser Ausführungsform zu der Grundstellung dadurch, als dass eine einsteckbare und wieder entfernbare Hülse 52 in dem Hebelmechanismus 48 vorgesehen ist, welche das Instrument 1 leicht öffnet, um ebenfalls den distalen Zangenabschnitt 46 gut reinigen zu können. Die Feder-Schenkel 16, 18 verlaufen in der Reinigungsstellung des Instruments 1 in einer ähnlichen Form wie die Griffe 10, 12, jedoch leicht nach proximal gestaucht, und liegen bzw. verlaufen insbesondere in Bereichen der Feder-Endabschnitte 20, 22 über eine Länge von 1/3 der Gesamtlänge parallel zueinander.

Um eine Ergonomie und Handhabbarkeit des Instruments weiter zu verbessern, sind an den äußeren Griffflächen 44 Handanschläge 50 vorgesehen, die an den Griffen 10, 12 senkrecht zur Instrumentenlängsachse seitlich nach außen vorstehen und in Ergreifung des Instruments eine Hand des Nutzers nach distal geometrisch begrenzen und schützen. Auch bieten sie einen Anschlag für eine Kraftaufbringung in distaler Richtung, etwa wenn das Instrument 1 entgegen eines Widerstands nach distal verschoben werden muss.

Fig. 6 zeigt in einer detaillierten Ansicht einer weiteren bevorzugten Ausführungsform eine Befestigung/einen Verbindungsbereich des Feder-Endabschnitts 16 auf der planen Kontaktauflagefläche 28 des Griffs 10 (hier exemplarisch nur die linke Seite des Instruments 1 dargestellt). Der Feder-Endabschnitt 16 ist mittels eines Klebers aufgeklebt, der medizintechnischen Anforderungen genügt und auch einer Sterilisation standhält.

Der erste podestförmige Vorsprung 24 weist distal eine plane Seitenfläche 29 und proximal eine geschwungene Seitenfläche 29 auf. Der Spaltwinkel α beträgt 80° und ein Winkel fußseitig des podestförmigen Absatzes entsprechend 100°. Eine Länge der Auflagekontaktfläche 28 in Richtung der Grifflängsachse gesehen beträgt 10mm. Diese (Mindest-)länge stellt eine ausreichende Auflagefläche für die Übertragung der Federkräfte sicher und bietet zudem eine ausreichende Klebefläche. Insbesondere beträgt die Größe der Kontaktauflagefläche 28 mindestens oder ungefähr 50mm².

Die in Fig. 6 gezeigte Befestigung zwischen dem Griff 10 und dem Feder-Schenkel 16 lässt sich in einfacher Weise auch auf die in Fig. 5 gezeigte Ausführungsform übertragen.

In einer weiteren bevorzugten Ausführungsform (nicht dargestellt) kann an dem podestförmigen Vorsprung zusätzlich noch ein aus und senkrecht zu der Kontaktauflagefläche vorstehender Pin vorgesehen sein, der in eine komplementäre Bohrung, ähnlich einer Stift-Bohrung-Verbindung, einsteht und den Federschenkel gegenüber dem Griff ausrichtet, so dass eine ungewollte Verdrehung der Federeinheit unterbunden wird.

### Bezugszeichenliste

- 1: Medizinisches Instrument
- 2: Erster Hebel
- 4: Zweiter Hebel
- 6: Scharnier
- 8: Griffabschnitt
- 10: Erstes Griffelement
- 12: Zweites Griffelement
- 14: Federeinheit
- 16: Erster Feder-Schenkel
- 18: Zweiter Feder-Schenkel
- 20: Erster Feder-Endabschnitt
- 22: Zweiter Feder-Endabschnitt
- 24: Erster podestförmiger Vorsprung
- 26: Zweiter podestförmiger Vorsprung
- 28: Kontaktauflagefläche
- 29: Seitenfläche
- 30: Höhe podestförmiger Vorsprung
- 32: Spaltbreite
- 34: Schraube
- 36: Schraubenlängsachse
- 38: Distale Feder-Kopplung
- 40: Nase
- 42: Gabel
- 44: äußere Grifffläche
- 46: distaler Zangenabschnitt
- 48: Hebelmechanismus
- 50: Handanschlag
- 52: Hülse
- S: Schwenkebene
- α: Spalt-Winkel

## Patentansprüche

1. Medizinisches, insbesondere chirurgisches, Instrument (1), mit zwei Griffelementen (10, 12), die schwenkbar zueinander gelagert sind, und einer Federeinheit (14), welche zwei Feder-Endabschnitte (20, 22) aufweist, die jeweils mit einem der beiden Griffelementen (10, 12) verbunden sind, so dass bei Verschwenken zumindest eines der beiden Griffelemente (10, 12) aus einer Grundstellung ein Zurückschwenken in die Grundstellung mittels der Federeinheit (14) durchführbar ist, wobei
die Federeinheit (14) dafür angepasst ist bei Verschwenken der Griffelemente (10, 12) eine im Wesentlichen gleichbleibende Federkraft bereitzustellen, insbesondere zumindest zweiteilig ausgebildet ist und einen ersten Feder-Schenkel (16) und einen zweiten Feder-Schenkel (18) aufweist, und
an zumindest einem der beiden Feder-Endabschnitte (20, 22) und/oder an zumindest einem der beiden Griffelemente (10, 12) ein podestförmiger Vorsprung (24, 26) ausgeformt ist, über den der Feder-Endabschnitt (20, 22) mit dem zugehörigen Griffelement (10, 12) starr verbunden ist, **dadurch gekennzeichnet,**
**dass** in zumindest der Grundstellung der zumindest eine Feder-Endabschnitt (20, 22) gegenüber dem zugehörigen Griffelement (10, 12) erhöht angeordnet ist und einen Abstand zu diesem aufweist, und
der podestförmige Vorsprung (24, 26) eine stirnseitige Kontaktauflagefläche (28) ausformt, über die der zumindest eine Feder-Endabschnitt (20, 22) an dem zugehörigen Griffelement (10, 12) befestigt ist.

2. Medizinisches Instrument (1) nach Anspruch 1, **dadurch gekennzeichnet, dass** die Federeinheit (14) zweiteilig in Form von zwei Blattfedern oder zwei Federstahldrähten ausgebildet ist, die über eine distale Gabel-Nasen-Verbindung (40, 42) oder eine distale Kugel-Pfannen-Verbindung miteinander gekoppelt sind.

3. Medizinisches Instrument (1) nach Anspruch 1 oder 2, **dadurch gekennzeichnet, dass** der podestförmige Vorsprung (24, 26) eine Höhe (30), insbesondere auf distaler Seite, von mindestens 1mm, bevorzugt von mindestens 2mm, ganz besonders bevorzugt von mindestens 4mm aufweist.

4. Medizinisches Instrument (1) nach Anspruch einem der vorstehenden Ansprüche, **dadurch gekennzeichnet, dass** der podestförmige Vorsprung (24, 26) blockförmig ausgebildet ist und insbesondere eine im Wesentlichen rechteckige Block-Grundfläche aufweist.

5. Medizinisches Instrument (1) nach einem der vorstehenden Ansprüche, **dadurch gekennzeichnet, dass** die Kontaktauflagefläche (28) des podestförmigen Vorsprungs (24, 26) mindestens 5mm² und/oder höchstens 1cm² beträgt und/oder plan ausgebildet ist.

6. Medizinisches Instrument (1) nach einem der vorstehenden Ansprüche, **dadurch gekennzeichnet, dass** ein Spalt-Winkel (α) zwischen einer dem zugehörigen Griffabschnitt (10, 12) zugewandten Seite des Feder-Endabschnitts (20, 22) und einer Seitenfläche (29) des podestförmigen Vorsprungs (24, 26), insbesondere einer distalen Seitenfläche (29), mindestens 20°, bevorzugt mindestens 40°, besonders bevorzugt über 65° und ganz besonders bevorzugt 90° beträgt.

7. Medizinisches Instrument (1) nach einem der vorstehenden Ansprüche, **dadurch gekennzeichnet, dass** der zumindest eine Feder-Endabschnitt (20, 22) über den podestförmigen Absatz (24, 26) mit dem zugehörigen Griffelement (10, 12) mittels einer Schraubverbindung und/oder stoffschlüssig, insbesondere mittels Verkleben oder Verschweißen oder Verlöten, verbunden ist.

8. Medizinisches Instrument (1) nach Anspruch 7, **dadurch gekennzeichnet, dass** im Falle der Schraubverbindung die Schraube (34) von Seiten des Feder-Endabschnitts (20, 22) in Richtung des Griffelements (10, 12) in einer Sacklochbohrung mit Innengewinde eingeschraubt ist, so dass das Griffelement (10, 12) an einer äußeren Grifffläche (44) kein Bohrloch aufweist oder dass die Schraube von Seiten der äußeren Grifffläche (44) des Griffelements (10, 12) in Richtung des Feder-Endabschnitts (20, 22) eingeschraubt ist, insbesondere in eine Sacklochbohrung mit Innengewinde.

9. Medizinisches Instrument (1) nach einem der vorstehenden Ansprüche, **dadurch gekennzeichnet, dass** der podestförmige Vorsprung (24, 26) ein Ausrichtungselement, insbesondere in Form eines vorspringenden Anschlags oder eines Pins aufweist, um den Feder-Endabschnitt (20, 22) gegenüber dem Griffelement (10, 12) auszurichten.

10. Medizinisches Instrument (1) nach einem der vorstehenden Ansprüche, **dadurch gekennzeichnet, dass** die einander zugewandten Außenkonturen und/oder Oberflächen von zumindest einem der beiden Griffelemente und dem zugehörigen Feder-Schenkel im Bereich des podestförmigen Vorsprungs (24; 26), insbesondere distal des podestförmigen Vorsprungs (24; 26), im Wesentlichen parallel zueinander verlaufen.

11. Medizinisches Instrument (1) nach einem der vorstehenden Ansprüche, **dadurch gekennzeichnet, dass** der podestförmige Vorsprung (24; 26) eine Länge in Richtung einer Längsachse des Feder-Endabschnitts (20; 22) von mindestens 5mm, bevorzugt mindestens 10mm, aufweist.

12. Medizinisches Instrument (1) nach einem der vorstehenden Ansprüche, **dadurch gekennzeichnet, dass** der podestförmige Vorsprung (24; 26) eine Höhe von maximal 5mm aufweist.

13. Medizinisches Instrument (1) nach einem der vorstehenden Ansprüche, **dadurch gekennzeichnet, dass** zumindest ein Feder-Endabschnitt (20; 22) derart mit dem zugehörigen Griffelement (10; 12) verbunden ist, dass die Federeinheit (14) sich nur in der Schwenkebene verformt.

## Claims

1. A medical, in particular surgical, instrument (1), having two gripping elements (10, 12) which are pivotably mounted relative to each other, and a spring unit (14) which has two spring end portions (20, 22), which are each connected to one of the two gripping elements (10, 12), so that when at least one of the two gripping elements (10, 12) is pivoted out of a base position, pivoting back into the base position can be carried out via the spring unit (14), wherein
the spring unit (14) is adapted to provide a substantially constant spring force when the gripping elements (10, 12) are pivoted, is in particular configured in at least two parts and has a first spring leg (16) and a second spring leg (18), and
a platform-shaped projection (24, 26) is formed on at least one of the two spring end portions (20, 22) and/or on at least one of the two gripping elements (10, 12), and via which the spring end portion (20, 22) is rigidly connected to the associated gripping element (10, 12), **characterized in that** the at least one spring end portion (20, 22) is arranged raised relative to the associated gripping element (10, 12) in at least the base position and has a distance therefrom,
the platform-shaped projection (24, 26) forms a front-face contact supporting surface (28) via which the at least one spring end portion (20, 22) is attached to the associated gripping element (10, 12).

2. The medical instrument (1) according to claim 1, **characterized in that** the spring unit (14) is formed in two parts in the form of two leaf springs or two spring steel wires, which are coupled to each other via a distal fork-nose connection (40, 42) or a distal sphere-pan connection.

3. The medical instrument (1) according to claim 1 or 2, **characterized in that** the platform-shaped projection (24, 26) has a height (30), in particular on the distal side, of at least 1 mm, preferably at least 2 mm, particularly preferably of at least 4 mm.

4. The medical instrument (1) according to one of the preceding claims, **characterized in that** the platform-shaped projection (24, 26) is block-shaped and in particular has a substantially rectangular block-base surface.

5. The medical instrument (1) according to one of the preceding claims, **characterized in that** the contact supporting surface (28) of the platform-shaped projection (24, 26) is at least 5 mm² and/or at most 1 cm² and/or is plane.

6. The medical instrument (1) according to one of the preceding claims, **characterized in that** a gap angle (α) between a side of the spring end portion (20, 22) facing the associated gripping portion (10, 12) and a side surface (29) of the platform-shaped projection (24, 26), in particular a distal side surface (29), is at least 20°, preferably at least 40°, particularly preferably more than 65° and very particularly preferably 90°.

7. The medical instrument (1) according to one of the preceding claims, **characterized in that** the at least one spring end portion (20, 22) is connected to the associated gripping element (10, 12) via the platform-shaped projection (24, 26) via a screw connection and/or is firmly bonded, in particular via gluing or welding or soldering.

8. The medical instrument (1) according to claim 7, **characterized in that,** in the case of the screw connection, the screw (34) is screwed into a blind hole bore with an inner thread from the side of the spring end portion (20, 22) in the direction of the gripping element (10, 12), so that the gripping element (10, 12) does not have a drilled hole on an outer gripping surface (44), or that the screw is screwed in from the side of the outer gripping surface (44) of the gripping element (10, 12) in the direction of the spring end portion (20, 22), in particular into a blind hole bore with an inner thread.

9. The medical instrument (1) according to one of the preceding claims, **characterized in that** the platform-shaped projection (24, 26) comprises an alignment element, in particular in the form of a protruding stop or a pin, in order to align the spring end portion (20, 22) with respect to the gripping element (10, 12).

10. The medical instrument (1) according to one of the preceding claims, **characterized in that** the mutually facing outer contours and/or surfaces of at least one of the two gripping elements and the associated spring leg run essentially parallel to each other in the region of the platform-shaped projection (24; 26), in particular distally of the platform-shaped projection (24; 26).

11. The medical instrument (1) according to one of the preceding claims, **characterized in that** the platform-shaped projection (24; 26) has a length in the direction of a longitudinal axis of the spring end portion (20; 22) of at least 5 mm, preferably at least 10 mm.

12. The medical instrument (1) according to one of the preceding claims, **characterized in that** the platform-shaped projection (24; 26) has a height of at most 5 mm.

13. The medical instrument (1) according to one of the preceding claims, **characterized in that** at least one spring end portion (20; 22) is connected to the associated gripping element (10; 12) in such a way that the spring unit (14) deforms only in the pivoting plane.

## Revendications

1. Instrument médical, en particulier chirurgical (1), avec deux éléments de préhension (10, 12), qui sont montés de manière à pouvoir pivoter l'un par rapport à l'autre, et une unité de ressort (14) qui présente deux sections d'extrémité à ressort (20, 22) qui sont reliées respectivement à l'un des deux éléments de préhension (10, 12), de sorte que lors du pivotement d'au moins l'un des deux éléments de préhension (10, 12) à partir d'une position initiale, un pivotement retour dans la position initiale peut être réalisé au moyen de l'unité de ressort (14), dans lequel
l'unité de ressort (14) est adaptée pour fournir une force élastique sensiblement constante lors du pivotement des éléments de préhension (10, 12), en particulier est conçue au moins en deux parties et présente une première branche à ressort (16) et une seconde branche à ressort (18), et
au niveau d'au moins l'une des deux sections d'extrémité à ressort (20, 22) et/ou au niveau d'au moins l'un des deux éléments de préhension (10, 12) une saillie en forme de plateforme est formée, par l'intermédiaire de laquelle la section d'extrémité à ressort (20, 22) est reliée de manière rigide à l'élément de préhension (10, 12) associé, **caractérisé en ce que** dans au moins la position initiale, la au moins une section d'extrémité à ressort (20, 22) est disposée de manière surélevée par rapport à l'élément de préhension (10, 12) associé et présente une certaine distance par rapport à celui-ci, et
la saillie en forme de plateforme (24, 26) forme une surface d'appui de contact (28) côté avant par l'intermédiaire de laquelle la au moins une section d'extrémité à ressort (20, 22) est fixée à l'élément de préhension (10, 12) associé.

2. Instrument médical (1) selon la revendication 1, **caractérisé en ce que** l'unité de ressort (14) est conçue en deux parties sous la forme de deux ressorts à lame ou deux fils d'acier à ressort qui sont couplés les uns aux autres par l'intermédiaire d'une liaison fourche-nez (40, 42) distale ou d'une liaison bille-cannelure distale.

3. Instrument médical (1) selon la revendication 1 ou 2, **caractérisé en ce que** la saillie en forme de plateforme (24, 26) présente une hauteur (30), en particulier du côté distal, d'au moins 1 mm, de préférence d'au moins 2 mm, le plus préférentiellement d'au moins 4 mm.

4. Instrument médical (1) selon l'une quelconque des revendications précédentes, **caractérisé en ce que** la saillie en forme de plateforme (24, 26) est conçue en forme de bloc et présente en particulier une surface de base sensiblement en forme de bloc rectangulaire.

5. Instrument médical (1) selon l'une quelconque des revendications précédentes, **caractérisé en ce que** la surface d'appui de contact (28) de la saillie en forme de plateforme (24, 26) est d'au moins 5 mm² et/ou au maximum de 1 cm² et/ou est conçue de manière plane.

6. Instrument médical (1) selon l'une quelconque des revendications précédentes, **caractérisé en ce que** un angle de fente (α) entre un côté tourné vers la section de préhension (10, 12) associée de la section d'extrémité à ressort (20, 22) et une surface latérale (29) de la saillie en forme de plateforme (24, 26), en particulier une surface latérale distale (29), est d'au moins 20°, de préférence d'au moins 40°, le plus préférentiellement supérieure à 65 ° et tout particulièrement le plus préférentiellement de 90°.

7. Instrument médical (1) selon l'une quelconque des revendications précédentes, **caractérisé en ce que** la au moins une section d'extrémité à ressort (20, 22) est reliée à l'élément de préhension (10, 12) associé par l'intermédiaire de l'avancée en forme de plateforme (24, 26) au moyen d'une liaison vissée et/ou par complémentarité de matière, en particulier au moyen d'un collage ou d'un soudage ou d'un brasage.

8. Instrument médical (1) selon la revendication 7, **caractérisé en ce que** dans le cas de la liaison vissée, la vis (34) est vissée depuis des côtés de la section d'extrémité à ressort (20, 22) en direction de l'élément de préhension (10, 12) dans un alésage borgne avec filetage intérieur, de sorte que l'élément de préhension (10, 12) ne présente aucun trou d'alésage au niveau d'une surface de préhension extérieure (44) ou **en ce que** la vis est vissée depuis des côtés de la surface de préhension extérieure (44) de l'élément de préhension (10, 12) en direction de la section d'extrémité à ressort (20, 22), en particulier dans un alésage borgne avec filetage intérieur.

9. Instrument médical (1) selon l'une quelconque des revendications précédentes, **caractérisé en ce que** la saillie en forme de plateforme (24, 26) présente un élément d'alignement, en particulier sous la forme d'une butée en saillie ou d'une épingle, afin d'aligner la section d'extrémité à ressort (20, 22) par rapport à l'élément de préhension (10, 12).

10. Instrument médical (1) selon l'une quelconque des revendications précédentes, **caractérisé en ce que** les contours extérieurs et/ou les surfaces tourné(e)s les un(e)s vers les autres d'au moins un des deux éléments de préhension et la branche à ressort associée s'étendent sensiblement parallèlement les un(e)s aux autres dans la zone de la saillie en forme de plateforme (24 ; 26), en particulier de manière distale par rapport à la saillie en forme de plateforme (24 ; 26).

11. Instrument médical (1) selon l'une quelconque des revendications précédentes, **caractérisé en ce que** la saillie en forme de plateforme (24 ; 26) présente une longueur en direction d'un axe longitudinal de la section d'extrémité à ressort (20 ; 22) d'au moins 5 mm, de préférence d'au moins 10 mm.

12. Instrument médical (1) selon l'une quelconque des revendications précédentes, **caractérisé en ce que** la saillie en forme de plateforme (24 ; 26) présente une hauteur de maximum 5 mm.

13. Instrument médical (1) selon l'une quelconque des revendications précédentes, **caractérisé en ce qu'**au moins une section d'extrémité à ressort (20 ; 22) est reliée à l'élément de préhension (10 ; 12) associé de telle sorte que l'unité de ressort (14) ne se déforme que dans le plan de pivotement.
